(19) Europäisches Patentamt
European Patent Office
Office européen des brevets

(11) **EP 2 453 004 B1**

(12) **FASCICULE DE BREVET EUROPEEN**

(45) Date de publication et mention de la délivrance du brevet:
**05.11.2014 Bulletin 2014/45**

(51) Int Cl.:
*C12M 1/107* *(2006.01)*    *C12M 1/16* *(2006.01)*
*C12P 5/02* *(2006.01)*

(21) Numéro de dépôt: **11188516.6**

(22) Date de dépôt: **09.11.2011**

(54) **Procédé et installation de méthanisation de matière organique à haute teneur en solides**

Verfahren und Anlage zur Methanisierung aus organischem Material mit hohem Feststoffanteil

Method and facility for anaerobic digestion of organic matter with high solid content

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.11.2010 BE 201000664**

(43) Date de publication de la demande:
**16.05.2012 Bulletin 2012/20**

(73) Titulaire: **Bertrand, Yves**
**1180 Bruxelles (BE)**

(72) Inventeur: **Bertrand, Yves**
**1180 Bruxelles (BE)**

(74) Mandataire: **Colens, Alain M.G.M. et al**
**Office Hanssens Colens**
**Square Marie Louise 40**
**Bte 19**
**1000 Bruxelles (BE)**

(56) Documents cités:
WO-A1-03/042117    WO-A1-2006/109588
DE-A1- 4 340 098    DE-A1-102007 029 748
US-A- 4 735 724

## Description

L'invention concerne un procédé et une installation de méthanisation de matière organique à haute teneur en solides, notamment encore que non exclusivement de déchets agricoles et d'élevage.

Etat de la technique

La méthanisation des matières organiques est un processus qui s'établit et se développe naturellement en l'absence d'oxygène, sous l'action de différentes espèces de bactéries anaérobies dont l'origine remonte à avant l'apparition d'une atmosphère d'oxygène sur notre planète. Ces bactéries sont présentes de manière quasi universelle sur terre, en particulier en symbiose dans le système digestif des animaux et de l'homme.

Les technologies utilisées pour mettre en oeuvre la méthanisation varient selon la nature du substrat à méthaniser et en particulier sa teneur en solides. La présente invention concerne les substrats à haute teneur en solides, c'est-à-dire supérieure à environ 15 %. Cette haute teneur en solides pose à l'intérieur des digesteurs de difficiles problèmes de transport que la technique connue a cherché à résoudre de différentes manières.

Les installations de méthanisation de substrats à haute teneur en solides sont essentiellement de deux types : la digestion semi-continue dans des cuves de fermentation infiniment mélangées, à une teneur en matière sèche d'environ 5 à environ 13 % et un temps de séjour de quelques semaines, et la digestion discontinue de fumiers à plus haute teneur en MS, avec recirculation du percolat et cuves ou conteneurs alimentés et vidés en alternance après un temps de séjour de quelques dizaines de jours. Dans le cas des digesteurs à la ferme, l'effluent de digestion est valorisé sur site par l'exploitant. Dans certains cas, une fraction solide de l'effluent subit un compostage aérobie final. Dans la majorité des cas, l'effluent n'est pas commercialisé. Le niveau d'investissement est élevé et malgré les aides à l'investissement et à la production d'énergie renouvelable, les temps de retour sur investissement sont dans la majorité des cas d'au moins de 10 anus : ces conditions n'ont pas permis un développement rapide de la filière.

Le document US 4,735,724 A décrit un digesteur verticale pour la méthanisation de matière organique à haute teneur en solides.

Il est utile de définir et distinguer ici deux paramètres importants de la mise en oeuvre de la méthanisation : le rendement et la productivité.

Le rendement de méthanisation s'exprime en $m^3$ de biogaz produit par kg de matière organique traitée ou en d'autres unités dérivées, par exemple en $m^3$ de $CH_4$ (en multipliant le volume de biogaz par sa teneur en $CH_4$) ou en kWh thermique (en multipliant le volume de $CH_4$ par son pouvoir calorifique) par kg de MO, de MS, de MV (matière volatile) ou de DCO (demande chimique en oxygène) traitées. Il dépend essentiellement de la nature du substrat, c'est-à-dire en gros du rapport C/N et du pH qui agissent sur l'équilibre entre les populations bactériennes. On peut agir sur la composition en mélangeant des matières premières d'origines différentes, ce qui permet de corriger le C/N et le pH. Le rendement de méthanisation ne dépend en pratique pas de la concentration en MO ni de la durée de réaction. En y mettant le temps, on peut épuiser totalement un substrat donné, dilué ou concentré, et obtenir chaque fois le même rendement de méthanisation.

Le rendement de méthanisation $\eta$ est défini par:

$$\eta = m^3{}_{biogaz}/kg_{MO}$$

ou les unités dérivées définies ci-dessus.

On peut déterminer le rendement $\eta$ d'un substrat donné par un simple essai discontinu, dans lequel on procède comme suit. On pèse et analyse la matière organique à tester, on l'introduit avec un inoculum dans un récipient fermé, maintenu à la température de test et équipé d'une sortie de gaz qui traverse un dispositif de comptage et d'analyse de la composition du gaz. On relève la production cumulée de gaz jusqu'à épuisement de la matière organique. A la fin de l'essai, les relevés de production et d'analyse du gaz produit permettent de calculer $\eta$ avec les résultats d'analyse de la matière organique. L'essai donne également une idée de la cinétique de la méthanisation de la matière organique à tester.

La productivité d'un méthaniseur ($m^3$ de biogaz par $m^3$ de digesteur et par unité de temps) est un paramètre lié à la fois au temps et à la quantité de substrat présente, et elle fait donc intervenir à la fois la cinétique (ou vitesse) de réaction et la concentration du substrat méthanisable. Pour augmenter la productivité d'un digesteur qui traite un substrat d'origine donnée, le premier paramètre sur lequel on peut agir est la température. On distingue trois plages: la plage psychrophile (5 ... 20°C), la plage mésophile (30 ... 40°C) et la plage thermophile (55 ... 65°C). A chaque plage correspondent des populations bactériennes spécifiques et des taux d'activité spécifiques à ces populations. Une augmentation de température agit sur la cinétique de la méthanisation (plus de $m^3$ de gaz produits par unité de temps).

A une température donnée et pour un substrat donné, un paramètre qui permet d'agir sur la productivité est la concentration en MO : toutes choses étant égales par ailleurs, la productivité d'un m³ de digesteur est évidemment d'autant plus haute que son contenu en MO est élevé.

La productivité s'exprime en m³ de biogaz par m³ de cuve et par unité de temps :

$$\pi = m^3{}_{biogaz} / m^3{}_{cuve}.t$$

ou en unité dérivées m³ de $CH_4$ ou kWh par m³ de cuve et par unité de temps.

Rendement $\eta$ et productivité sont liés par la concentration $\gamma$ exprimée par exemple en $kg_{MO}$ par m³ de cuve :

$$\gamma = kg_{MO}/m^3{}_{cuve}$$

Si dans la relation

$$\pi = m^3{}_{biogaz} / m^3{}_{cuve}.t$$

on multiplie haut et bas par $kg_{MO}$ :

$$\pi = (m^3{}_{biogaz}.kg_{MO})/(m^3{}_{cuve}.\ kg_{MO}.t)$$

on obtient en regroupant différemment :

$$\pi = (m^3{}_{biogaz}/kg_{MO}).(kg_{MO}/m^3{}_{cuve}.t)$$

et en substituant par $\eta = m^3{}_{biogaz}/kg_{MO}$ et $y = kg_{MO}/m3$ cuve :

$$\pi = \eta.\gamma/t$$

et si on pose $\gamma/t = \kappa$ (débit entrant de MO) :

$$\pi = \eta.\kappa$$

En d'autres termes, la productivité est la dérivée par rapport au temps du produit du rendement de méthanisation et de la concentration en MO ou, exprimé autrement, le produit du rendement de méthanisation par le débit entrant de matière organique.

En plus de la teneur en matière organique de la masse en fermentation, un autre paramètre joue un rôle essentiel sur le rendement et la productivité de la méthanisation : les conditions de mélange et d'homogénéité.

Il existe deux cas extrêmes de mélange : le fermenteur infiniment mélangé et le fermenteur piston ou stratifié (en anglais "plug-flow").

Le contenu de la cuve d'un fermenteur infiniment mélangé est brassé en permanence ou par intermittences pendant la fermentation. Si ce fermenteur travaille en mode semicontinu, c'est-à-dire en étant alimenté par exemple une fois par jour d'un volume de matière première qui correspond au temps de séjour fixé (par exemple pour un temps de séjour de 25 jours, ce volume correspondra à 1/25 du volume V de la cuve), avec extraction d'un volume équivalent, de manière à maintenir constant le niveau dans la cuve), le contenu du fermenteur est statistiquement constitué de 25 fractions qui ont séjourné de 25 à 1 jour dans la cuve - dans notre cas une fraction de 1/25 V qui y a séjourné 25 jours, une autre de 1/25 V qui n'y a séjourné que 24 jours, et ainsi de suite jusqu'à une fraction de 1/25 V qui n'y a séjourné qu'un jour. Si on suppose en première analyse que la méthanisation progresse linéairement avec le temps de séjour dans le fermenteur, chaque fraction de 1/25 V subit chaque jour 1/25 de la dégradation anaérobie totale.

Ainsi, la dégradation anaérobie totale est formée de la somme de 25 termes (V/25) * (n/25) avec n = 1,..., 25.

Cette somme vaut

(V/25)*[(1 + 2 + 3 + ... + 25)/25]

soit 325/625 = 0,52

ou de façon plus générale

(1/n) * [(1 + 2 + 3 + ... + n) /n]

soit

$$[\Sigma \; (1...n)]/n^2$$

qui tend asymptotiquement vers 0,50 lorsque n augmente.

On peut donc conclure qu'une méthanisation semi-continue ou continue conduite en conditions de mélange homogène dans la cuve de fermentation ne permet d'épuiser que la moitié du potentiel de méthanisation de la matière introduite.

Ce mode de mise en oeuvre de la méthanisation est donc le pire à envisager, puisqu'il ne permet de dégrader que de l'ordre de la moitié du potentiel de méthanisation initialement présent. Il est pourtant largement utilisé sur des matières organiques à haute teneur en solides mais diluées pour résoudre les problèmes de transport dans les digesteurs. Pour contrer la flottation des matières fibreuses dans ces digesteurs, on assure un brassage vigoureux de la masse diluée, ce brassage étant lui-même gros consommateur d'énergie.

Voyons ensuite l'autre extrême, le fermenteur piston idéal. Dans ce fermenteur, l'alimentation avance par strates successives entre l'entrée et la sortie, chaque strate restant théoriquement distincte de la précédente et de la suivante. Dans un tel mode de conduite de la fermentation, la sortie est complètement épuisée de son potentiel de méthanisation: on y obtient donc le rendement de méthanisation le plus élevé qui, toutes autres choses étant égales par ailleurs, peut atteindre le double de celui d'un digesteur infiniment mélangé.

Enfin, à une température donnée et pour une matière organique donnée, l'activité bactérienne est le produit du nombre de bactéries par l'activité de chacune sur le substrat, qui elle-même est fonction entre autres de l'accessibilité du substrat : un certain niveau de brassage est donc avantageux par son action sur l'interface de contact entre les bactéries et le substrat, surtout si ce dernier présente une forte composante solide.

Ce paramètre du brassage est donc un élément essentiel du bon déroulement de la fermentation. Avec l'établissement et le maintien d'une haute activité bactérienne, c'est la manière dont ce paramètre est pris en compte qui détermine les différents modes de mise en oeuvre de la méthanisation. Dans le cas de la méthanisation de substrats à haute teneur en matière solide (supérieure à environ 15 %), on observe en effet la formation de poches de gaz dans la masse en fermentation et par corollaire, le maintien de zones compactes de substrat peu accessibles aux bactéries. Le brevet européen EP 1 397 482 B1 traite ce problème et cherche à le résoudre dans un digesteur piston à avancement gravitaire du substrat en renvoyant en tête du digesteur une partie du substrat extrait tout en réalisant un équilibrage des densités du substrat extrait et du substrat frais, mais avec une importante mise en oeuvre en appareils qui renchérit le coût de l'installation.

Un brassage intime de la masse en fermentation entraîne cependant une homogénéisation qui peut avoir pour conséquence un "rétro-mélange" qui mêle des fractions peu digérées à d'autres où la digestion a progressé davantage. Quand bien même ce brassage intime de toutes les fractions serait possible dans des conditions de forte teneur en solides, cela ramène les conditions du processus à celles d'un digesteur infiniment mélangé qui, nous l'avons exposé plus haut, ne permettent pas d'épuiser à plein le potentiel de méthanisation du substrat.

Pour assurer un bon brassage et une bonne activité bactérienne dans des conditions de fermentation en digesteur piston à déplacement gravitaire, la demande de brevet EP 1 930 404 A1 propose de recycler une partie de substrat soutirée à une hauteur intermédiaire en la mélangeant à du substrat frais, de manière à inoculer des bactéries dans ce dernier. On laisse la fermentation se terminer dans la partie inférieure du digesteur avant d'extraire le substrat digéré. Cependant, ce recyclage nécessite lui aussi une importante mise en oeuvre en appareils qui renchérit le coût de l'installation. D'autre part, cette technologie revient à la combinaison d'une digestion en conditions de mélange homogène au-dessus du niveau de soutirage de la matière recyclée et d'une digestion en conditions d'avancement piston dans la partie du digesteur située en dessous du niveau de soutirage de la matière recyclée.

Présentation de l'invention

Compte tenu de ce qui vient d'être exposé, l'invention propose un procédé et une installation de méthanisation de matière organique à haute teneur en matières solides qui soient plus performants à la fois en termes de rendement de méthanisation et en termes de productivité et de simplicité de l'installation, de manière à la fois à abaisser le coût de l'investissement et à augmenter le revenu qu'il permet.

L'invention résout ce problème avec le procédé défini dans la revendication indépendante de procédé et avec l'installation définie dans la revendication indépendante de dispositif. Les revendications subordonnées définissent des modes de réalisation avantageux du procédé et de l'installation.

Le procédé de fermentation anaérobie de matière organique à haute teneur en solides selon l'invention, conduit dans une cuve de fermentation étanche vis-à-vis de l'air ambiant, en conditions de stratification et d'avancement vertical du haut en bas dans la cuve de fermentation sous l'action de la gravité, est caractérisé par un brassage de la masse en fermentation par alternance de montées en pression du gaz produit et de délestages brusques de la pression du gaz produit, cette alternance étant obtenue par soutirage cadencé du gaz produit.

Bien qu'il existe déjà dans l'état de la technique des systèmes et des procédés qui recourent à une ou plusieurs des techniques utilisées dans la présente invention, aucun de ces systèmes et procédés de l'état de la technique n'y recourt de la manière proposée par l'invention pour en réunir les avantages.

Il existe en effet des digesteurs travaillant en mode "piston" sur des déchets à haute teneur en matières solides, mais si la direction d'avancement est globalement horizontale et non verticale, au lieu de tirer profit de la gravité, ils doivent recourir à des mécanismes complexes de transport interne dans le digesteur. De plus, la sédimentation gravitaire des fractions lourdes a lieu dans une direction perpendiculaire à la direction d'avancement de la masse en fermentation et ces fractions potentiellement perturbatrices ne sont pas extraites naturellement avec la matière fermentée comme dans le procédé et l'installation selon l'invention.

Il existe des procédés de méthanisation qui réinjectent à la base de la masse en fermentation une partie du gaz produit pour assurer un brassage de la masse en fermentation, mais ce brassage par injection de gaz est consommateur d'énergie alors que le brassage utilisé dans l'invention n'en consomme aucune.

Enfin, nous l'avons mentionné plus haut, il existe des procédés de méthanisation qui renvoient en tête du digesteur une partie du substrat extraite à un niveau intermédiaire et mélangée à du substrat frais, de manière à maintenir dans le digesteur une plus grande partie des bactéries qui s'y sont développées et ainsi renforcer l'activité méthanogène. Cependant, la mise en oeuvre en appareils alourdit le coût de l'installation et l'énergie consommée pour faire fonctionner ces appareils dégrade leur rendement énergétique global.

Description détaillée de modes de réalisation préférés de l'invention

Un mode de réalisation actuellement préféré de l'invention va maintenant être décrit à titre d'exemple et illustré dans les dessins annexés, dans lesquels :

la figure 1 représente un schéma de principe du procédé selon l'invention,

la figure 2 représente en coupe verticale schématique une installation de fermentation anaérobie de matière organique à haute teneur en solides selon l'invention,

la figure 3 représente schématiquement la même installation en élévation découpée pour en montrer plus en détail certains éléments,

la figure 4 représente schématiquement la manière dont la pression du gaz produit dans la cuve de fermentation évolue sous l'action des systèmes 19 de brassage par le gaz produit selon l'invention,

la figure 5 représente schématiquement un exemple de réalisation d'un système 19 de brassage par le gaz produit selon l'invention et

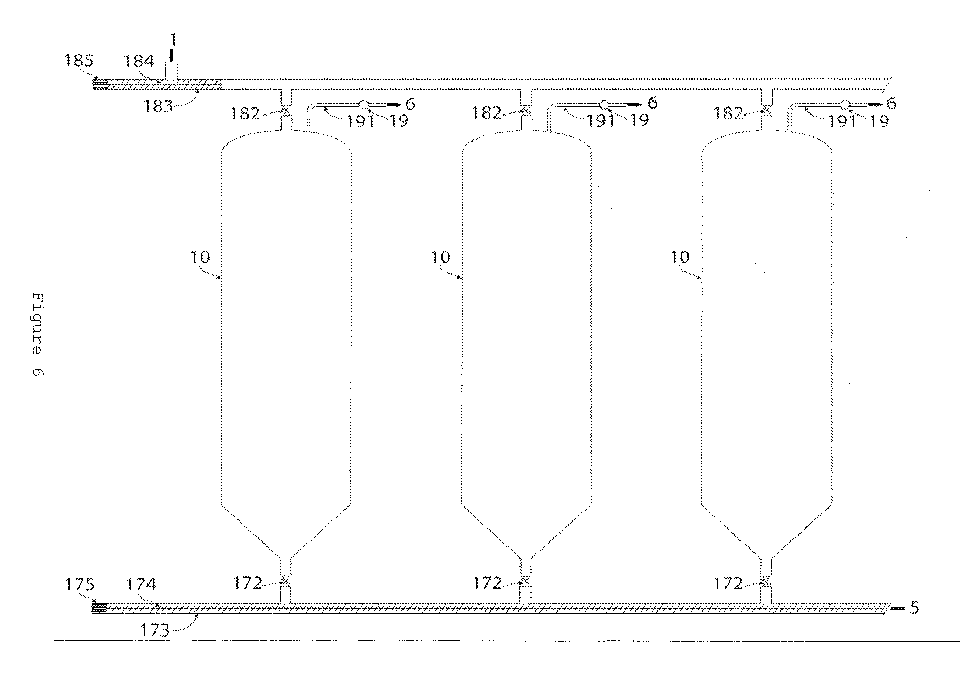

la figure 6 représente schématiquement une installation constituée de la mise en parallèle de plusieurs des modules représentés dans la figure 2.

Dans toutes les figures, des parties identiques, similaires ou assurant la même fonction ont été dotées des mêmes références numériques.

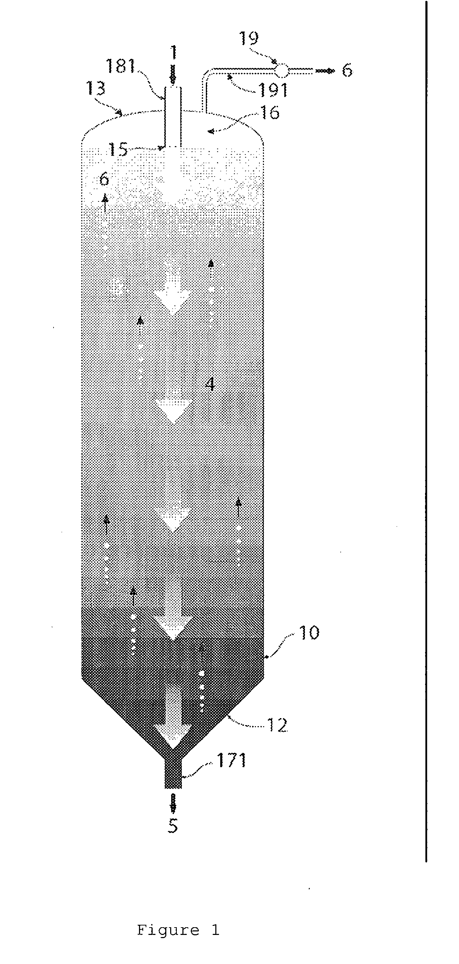

Le principe du procédé selon l'invention est représenté dans la figure 1. Le procédé est conduit dans au moins une cuve de fermentation anaérobie 10 qui présente la forme d'une virole verticale fermée dans le haut par un dôme supérieur 13 traversé par une tubulure d'alimentation 181 et dans le bas par un cône inférieur 12 dont la pointe est tournée vers le bas et est traversée par une tubulure d'extraction 171. Les détails de construction de la cuve seront donnés plus loin ; à ce stade de la description, indiquons que les proportions de la cuve sont importantes et que sa hauteur représente de préférence de l'ordre de 3 à 6 fois son diamètre.

La cuve 10 posée verticalement, dôme dans le haut et cône dans le bas, est alimentée par le haut en matière organique 1 à haute teneur en matières solides qui descend progressivement du dôme 13 vers le cône 12 sous l'action de la gravité, comme matière en fermentation 4, pour être extraite par la tubulure d'extraction 171 en tant que matière fermentée 5. En régime, le débit massique d'alimentation en matière organique 1 à haute teneur en matières solides est égal stricto sensu à la somme du débit massique d'extraction de matière fermentée 5 et du débit massique de gaz de fermentation 6 (biogaz), de sorte que la masse totale que contient la cuve est constante.

Ce mode de conduite de la fermentation est une caractéristique essentielle de l'invention et il présente deux cas limite : la conduite discontinue stricte, dans laquelle la cuve est remplie en une fois, la matière introduite étant maintenue en fermentation anaérobie pendant un temps de séjour prédéterminé et étant ensuite vidée de son contenu fermenté, et la conduite continue stricte, dans laquelle le débit massique d'alimentation est constant, correspond à un temps de séjour prédéterminé et est égal à la somme du débit massique d'extraction et du débit massique de production de biogaz. Entre ces deux extrêmes, on trouve la conduite semi-continue dans laquelle une quantité de matière fermentée 5 qui correspond au temps de séjour visé est extraite chaque jour en une ou plusieurs fois, une quantité de matière organique 1 qui correspond au maintien d'un poids constant dans la cuve compte tenu de la masse de biogaz extraite est introduite chaque jour en une ou plusieurs fois après chaque extraction.

Au cours de sa descente progressive dans la cuve 10, la matière en fermentation 4 subit les différentes étapes de la dégradation par les bactéries anaérobies.

Le biogaz 6 produit au cours de cette dégradation est extrait par un conduit 191. Ce biogaz est dégagé dans la masse en fermentation 4, mais son extraction par le conduit d'extraction 191 est contrôlée en fermant le conduit 191 jusqu'à ce que la pression du biogaz 6 dans la cuve 10 atteigne une valeur prédéterminée, et en ouvrant le conduit lorsque cette pression prédéterminée et réglable est atteinte. Cette alternance de montées progressives et de détentes brusques de la pression provoque un effet de soulèvement-gonflement de la masse en fermentation 4, mais contrairement à ce qui se produit lorsque cet effet est pratiqué dans un milieu liquide, elle ne perturbe pas la stratification de la matière en fermentation 4 qui descend progressivement dans la cuve 10.

On obtient ainsi non seulement un fermenteur stratifié ou piston qui exclut tout rétro-mélange entre la matière fermentée 5 extraite et la matière organique 1 introduite, mais de plus un fermenteur qui bénéficie d'un brassage qui ne perturbe pas la stratification de la matière en fermentation 4 dans la cuve de fermentation 10 et qui s'exerce à contre-courant dans la masse en déplacement.

La combinaison de caractéristiques que propose l'invention, à savoir :

- un digesteur allongé dont la direction longitudinale est disposée à la verticale,
- alimenté en matière organique à haute teneur en matières solides,
- un avancement vertical descendant de la masse en fermentation sous l'action de la gravité,
- un brassage obtenu par alternance de montées progressives et de détentes brusques de la pression du biogaz produit avec un avancement vertical ascendant du biogaz produit à contre-courant de l'avancement vertical descendant de la matière en fermentation,

contribue de manière nouvelle et décisive à l'optimisation de la mise en oeuvre de la fermentation.

A la différence des systèmes de l'état de la technique, tous ces effets sont obtenus sans autre dépense en énergie mécanique que celle nécessaire à l'apport de matière organique dans le fermenteur, parce qu'ils sont obtenus sans devoir prévoir un système tel qu'une vis sans fin ou autre pour faire avancer la masse en fermentation, pas davantage qu'un système qui comprime le biogaz avant de le réinjecter dans la masse en fermentation par le bas du fermenteur. Le maintien strict de conditions d'avancement en piston garantit également l'épuisement complet du potentiel de méthanisation du substrat et la suppression du renvoi en tête d'une partie de la masse en fermentation.

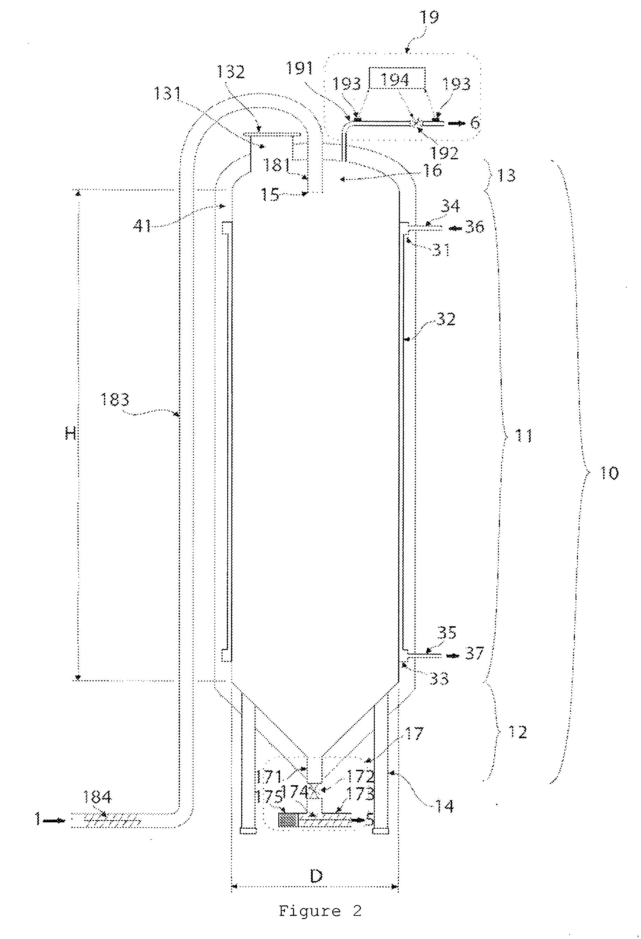

La figure 2 représente plus en détail un mode de réalisation du fermenteur anaérobie selon l'invention, dans lequel le procédé selon l'invention est mis en oeuvre. La figure 2 représente une coupe verticale, passant par l'axe longitudinal central, du fermenteur anaérobie selon l'invention. Ce fermenteur, réalisé par exemple en acier, est constitué d'une cuve 10 constituée d'une virole cylindrique 11, par exemple circulaire, posée verticalement sur le sol par des pieds 14 et fermée dans le haut par un dôme 13 et dans le bas par un cône 12 dont la pointe est tournée vers le bas. Une tubulure d'extraction orientée à la verticale traverse la pointe du cône 12. L'angle d'ouverture du cône sera de préférence de 45°, mais d'autres valeurs de cet angle sont envisageables. Le dôme supérieur 13 est traversé par une tubulure d'alimentation 181 et par une tubulure de sortie de gaz 191. La tubulure d'alimentation 181 pénètre par le haut dans la cuve de fermentation anaérobie 10 jusqu'à un niveau bas 15 qui définit une partie 16 du volume de la cuve 10 exempt de matière en fermentation. Un trou d'homme 131 fermé par une vitre 132 peut être prévu dans le dôme pour donner accès à l'intérieur de la cuve et permettre d'en observer le contenu. La hauteur intérieure H de la virole cylindrique 11 représente de préférence de 3 à 6 fois son diamètre intérieur. Ces proportions assurent un effet de stratification de la masse en fermentation suffisant pour exclure tout court-circuit d'écoulement ou rétro-mélange entre l'entrée et la sortie.

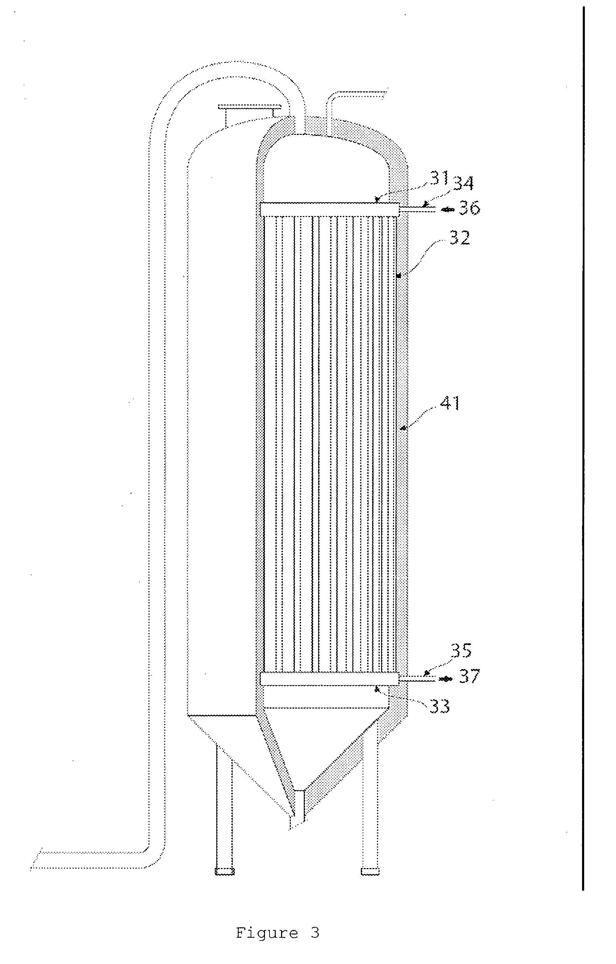

La face extérieure de la virole 11 est dotée d'un circuit de chauffage qui assure le maintien de la température de fermentation T, et d'une isolation thermique extérieure 41 qui limite les pertes thermiques de la cuve de fermentation et qui couvre également le dôme 13 et le cône 12. Le circuit de chauffage est par exemple constitué d'un collecteur supérieur 31, d'un collecteur inférieur 33 et de tubes de répartition 32. Tous ces éléments, par exemple réalisés en acier, sont fixés sur la paroi extérieure de la virole 11 de manière thermiquement conductrice, par exemple par soudage. Le fluide caloporteur, de préférence de l'eau 36, chauffée par une partie de l'énergie produite par la combustion du gaz produit 6 ou autrement, est apportée par exemple dans le collecteur supérieur 31 par une tubulure 34 et est reprise par une tubulure 35 raccordée au collecteur inférieur 33 pour être renvoyée en 37 vers le système de chauffage. Une régulation de température non représentée assure la gestion du système de chauffage qui maintient une température de fermentation appropriée, par exemple d'environ 35 à 39°C (conditions de fermentation mésophile) ou d'environ 55 à 65°C (conditions de fermentation thermophile). Une isolation thermique 41 recouvre l'ensemble de la cuve ainsi que les collecteurs 31, 33 et tubes de répartition de chauffage 32. La figure 3 donne une autre représentation de la cuve 10 équipée de son système de chauffage et de son isolation thermique. La conception, le dimensionnement et la réalisation de ces systèmes de chauffage et d'isolation sont bien connus dans la technique et ne font pas partie de la présente invention.

D'un point de vue industriel, il est intéressant de pouvoir fabriquer en usine la cuve de fermentation 10 et ses systèmes annexes sous la forme de modules de volume défini. Le volume de ces modules est limité par les restrictions au transport entre l'usine et le site d'installation. Pour un diamètre intérieur de 3 m, une hauteur de virole de 10 m (ces valeurs sont compatibles avec les dimensions maximales d'un transport par camion) et un angle du cône de 45°, le volume utile (hors dôme) d'un module est donné par la relation :

$$V = \pi(D^2/4)*H + 1/3[(\pi D^2/4)(D/2)]$$

soit 74,22 m$^3$ pour H = 10 m et D = 3 m.

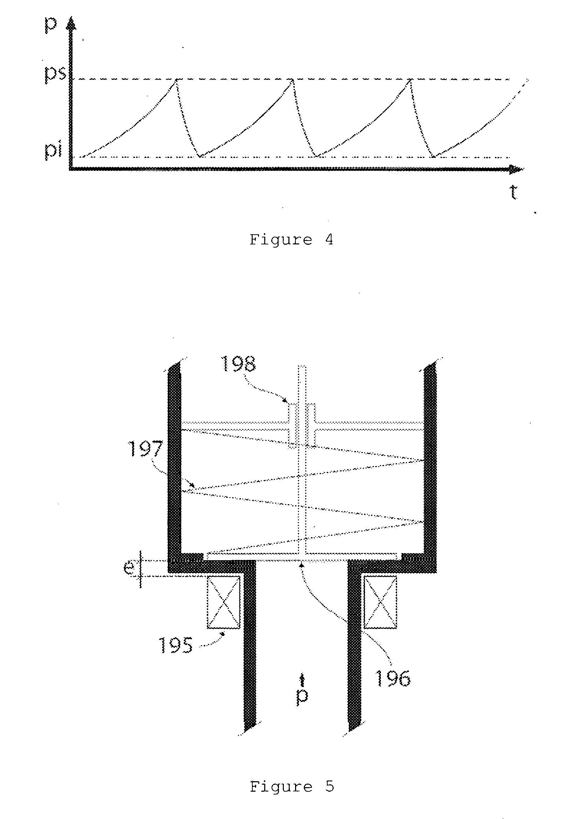

Selon les besoins, on prévoira donc plusieurs de ces modules qui seront alors raccordés en parallèle entre un système d'extraction et un système d'alimentation communs (voir figure 4). A titre indicatif, une cuve de 75 m$^3$ de volume utile permet de traiter de l'ordre de 1 000 tonnes/an de matière organique à 35 % de matière sèche.

Un système 17 d'extraction de la matière fermentée 5 est raccordé à la tubulure d'extraction 171. De tels systèmes sont bien connus dans la technique et ils ne sont donc pas couverts par l'invention. L'exemple de réalisation de ce système représenté dans la figure 2 comprend .

- une vanne d'extraction 172,
- un conduit d'extraction 173 doté d'une vis sans fin 174 conduisant à la poursuite du traitement de la matière fermentée et
- un moteur 175 qui entraîne la vis d'extraction,

mais il pourrait être remplacé par un autre type de système d'extraction, par exemple une simple fosse ou une bande transporteuse.

Un système d'alimentation 18 est raccordé à la tubulure d'alimentation 181. De nouveau, de tels systèmes sont connus, et ils ne sont pas inclus dans l'invention. L'exemple de réalisation de ce système représenté dans la figure 2 comprend .

- la tubulure d'alimentation 181
- un conduit d'alimentation 183 et
- un système de transport 184.

Le système de transport 184 peut être tout système connu et éprouvé de refoulement de matière dense et fibreuse (par exemple du fumier). Le système représenté à titre d'exemple dans la figure 2 est également une vis sans fin, mais d'autres systèmes sont possibles.

La cuve est dotée d'un système de commande d'alimentation conçu pour effectuer les actions suivantes : activation du système d'extraction 17 de manière à extraire à intervalles donnés une masse donnée de matière fermentée 5 de la cuve de fermentation 10, suivie par l'activation du système d'alimentation 18 de manière à introduire une masse de matière organique 1 à haute teneur en solides dans la cuve de fermentation 10. La masse extraite chaque jour de la cuve de fermentation 10 sera celle qui correspond au temps de séjour défini de la masse en fermentation 4 dans la cuve de fermentation 10 et la masse de matière organique 1 à haute teneur en solides apportée chaque jour dans la cuve de fermentation 10 sera la quantité qui permet de maintenir une quantité constante de masse en fermentation 4 dans la cuve de fermentation 10. Dans la phase de démarrage, lorsque la cuve de fermentation 10 est remplie jour après jour d'une quantité qui correspond au temps de séjour visé, seul le système d'alimentation est activé journellement.

Pour autant que le système de commande permette de contrôler l'alimentation et l'extraction de matière dans et hors du digesteur de manière à conserver un temps de séjour donné de la masse en fermentation dans la cuve 10 et de la maintenir sensiblement constante, il peut être réalisé de manières très variées et être basé par exemple sur le maintien d'un niveau constant de matière dans la cuve 10 (avec une ou plusieurs sondes de niveau) ou sur le maintien d'un poids constant de matière dans la cuve 10 (avec une ou plusieurs sondes de charge sous les pieds 14 de la cuve 10). Les signaux délivrés par ces sondes peuvent alors commander le fonctionnement du système d'extraction 17 et du système d'alimentation 18. Ces systèmes sont bien connus dans la technique et ne sont pas couverts par l'invention.

Un composant essentiel de l'installation de fermentation anaérobie selon l'invention est son système 19 de brassage par le gaz produit 6, dont un exemple de réalisation est représenté schématiquement dans la figure 2. Il est fondamentalement constitué de la tubulure 191 de sortie du gaz produit 6, d'une vanne de fermeture/ouverture 192, de sondes de pression 193 disposées en amont et en aval de la vanne 192 dans la direction de sortie du gaz produit 6 et d'un régulateur de pression 194. On entre dans le régulateur de pression 194 deux valeurs de pression, une valeur supérieure et une valeur inférieure. La valeur supérieure de pression est comprise entre environ 100 et environ 500 mbar et la valeur inférieure de pression est sensiblement la pression qui règne dans la réserve de biogaz produit 6 (non représentée), par exemple un ballon souple de stockage de biogaz. Le régulateur de pression ouvre alors la vanne 192 normalement fermée lorsque la pression mesurée par la sonde de pression 193 située en amont de la vanne 192 atteint la valeur supérieure et la referme lorsqu'elle est redescendue à la valeur mesurée par la sonde 193 située en aval de la vanne 192.

Une autre variante de réalisation du système 19 de brassage par le gaz produit 6 utilise une seule sonde de pression 193 dont les signaux de pression sont envoyés au régulateur qui ouvre ou ferme la vanne 192 de manière à assurer le soutirage cadencé du gaz.

Dans une autre variante de réalisation du système 19 de brassage par le gaz produit 6, la vanne 192 est un clapet magnétique dont la force de l'aimant permanent 195 maintient un organe de fermeture 196 apte à coulisser dans une douille de guidage 198 en position de fermeture tant que la pression n'atteint pas une valeur prédéterminée $p_s$ qui peut être réglée en ajustant la distance e entre l'aimant 195 et l'organe de fermeture 196. Lorsque cette valeur est atteinte, la poussée du gaz éloigne suffisamment l'organe de fermeture 196 de l'aimant 195 pour que l'organe de fermeture 196 soit maintenu ouvert par l'effet du débit du gaz 6 qui s'échappe. Lorsque le débit de gaz 6 diminue et que donc sa pression s'approche de la pression $p_i$ qui règne dans une réserve de gaz située en aval et constituée d'un ballon souple ou d'un gazomètre, l'organe de fermeture 196 se rapproche à nouveau de l'aimant sous l'effet de son poids ou facultativement d'un ressort 197 taré de manière appropriée.

L'effet de cette alternance de montées lentes et de délestages brusques de la pression du biogaz 6 dans la cuve de fermentation 10 a été observé dans un récipient de fermentation transparent : la masse en fermentation se soulève et se gonfle sous l'effet de la détente, ce qui réorganise les cavités et cheminées remplies de gaz qui se sont formées dans la masse en fermentation 4 pendant les intervalles de fermeture du conduit 191 de sortie de gaz, sans pour autant perturber la stratification de la masse en fermentation 4. Cette réorganisation a pour effet de renouveler régulièrement les interfaces de contact entre les bactéries et la masse en fermentation 4 et de maintenir un haut niveau d'activité bactérienne dans la masse en fermentation.

Pour le reste, l'installation de fermentation anaérobie selon l'invention comporte les composants classiques d'une telle installation, par exemple :

- un système de préparation de la charge (hachage, mélange, éventuellement fermenteur aérobie),
- un système de stockage du gaz produit (ballon ou gazomètre),
- un système d'épuration du biogaz (entre autres séparation du $H_2S$),
- un système de valorisation du gaz (chaudière, groupe de cogénération de chaleur et d'électricité) et
- un système de séchage de la matière fermentée alimenté par exemple par la chaleur produite par chaudière ou groupe de cogénération.

Ces systèmes annexes sont bien connus dans la technique et ne sont pas inclus dans l'invention.

Possibilités d'application industrielle

L'invention peut être appliquée dans le domaine de l'agriculture, de l'élevage et dans le secteur agroalimentaire chaque fois que de la matière organique à haute teneur en matières solides ou susceptible d'être mélangée à d'autres matières organiques de manière à obtenir un mélange de matière organique à haute teneur en matière solide peut ou doit être traitée pour réduire son pouvoir polluant et/ou pour produire de l'énergie et/ou de l'engrais. Elle permet cette réduction de manière très simple et plus efficace qu'avec les systèmes existants, à un coût d'investissement plus bas et en valorisant mieux à la fois le potentiel énergétique et le potentiel agronomique des déchets traités : elle exploite mieux qu'avec les systèmes infiniment mélangés classiques le potentiel de production de biogaz des déchets traités, et comme elle produit une matière fermentée à haute teneur en solides, elle facilite son séchage et permet ainsi de stocker, de transporter et de commercialiser l'effluent solide de méthanisation plus aisément que les effluents de la fermentation anaérobie classique en phase liquide.

De nombreuses variantes et modifications peuvent être apportées à l'invention sans pour autant sortir de sa portée qui est définie par les revendications qui suivent.

Liste des références alphanumériques

1 Matière organique à haute teneur en solides

2 Matière organique fibreuse

3 Matière organique à basse teneur en solides

4 Masse en fermentation

5 Matière fermentée quittant la cuve de fermentation

6 Gaz produit

10 Cuve de fermentation anaérobie

11 Virole

12 Cône inférieur

13 Dôme supérieur

14 Pieds

H Hauteur de la virole 11

D Diamètre de la section transversale de la virole

15 Niveau bas de la tubulure d'alimentation dans la cuve

16 Volume de la cuve exempt de matière organique

17 Système d'extraction

18 Système d'alimentation

19 Système de brassage

31 Collecteur supérieur de chauffage

32 Tubes de répartition de chauffage

33 Collecteur inférieur de chauffage

| 34 | Tubulure |
| --- | --- |
| 35 | Tubulure |
| 36 | Entrée fluide de chauffage |
| 37 | Sortie fluide de chauffage |
| 41 | Isolation thermique extérieure |
| 131 | Trou d'homme |
| 132 | Vitre |
| 171 | Tubulure d'extraction |
| 172 | Vanne d'extraction |
| 173 | Conduit d'extraction |
| 174 | Vis sans fin |
| 175 | Moteur |
| 181 | Tubulure d'alimentation |
| 183 | Conduit d'alimentation |
| 184 | Système de transport |
| 191 | Tubulure de sortie du gaz produit |
| 192 | Vanne de sortie de gaz |
| 193 | Sonde de pression |
| 194 | Régulateur de pression |
| 195 | Aimant permanent |
| 196 | Organe de fermeture |
| 197 | Ressort |
| 198 | Douille de guidage |
| $e$ | Entrefer entre l'aimant 195 et l'organe de fermeture 196 |
| $\theta$ | Temps de séjour |
| $T$ | Température de fermentation anaérobie |
| r) | Rendement de méthanisation |
| $\pi$ | Productivité de l'installation |

**Revendications**

1. Procédé de fermentation anaérobie de matière organique à haute teneur en solides, par recours à une fermentation anaérobie de la matière organique dans une cuve de fermentation (10) étanche vis-à-vis de l'air ambiant, en conditions de stratification et d'avancement vertical du haut en bas dans la cuve de fermentation (10) sous l'action de la gravité,
**caractérisé en ce que**
la masse en fermentation (4) est brassée par alternance de montées en pression du gaz produit (6) et de délestages brusques de la pression du gaz produit (6) et
**en ce que** cette alternance est obtenue par soutirage cadencé du gaz produit (6).

2. Procédé selon la revendication 1, **caractérisé en ce que** la teneur en solides de la matière organique (1) est comprise de préférence entre 15 et 50 % en poids.

3. Procédé selon la revendication 1, **caractérisé en ce que** la matière organique (1) est fermentée à une température comprise de préférence dans une plage de 35 à 40°C ou dans une plage de 55 à 65°C.

4. Procédé selon l'une ou plusieurs des revendications 1 à 3, **caractérisé en ce qu'**en régime permanent, une quantité de matière fermentée (5) égale à la masse contenue dans la cuve de fermentation divisée par le temps de séjour ($\theta$) de la masse en fermentation (4) dans la cuve de fermentation (10) est extraite chaque jour en une ou plusieurs passes d'extraction de la cuve de fermentation (10), le poids de la quantité de matière organique (1) introduit lors de chaque passe d'alimentation étant tel qu'il ramène le niveau ou le poids de la quantité de matière que contient la cuve de fermentation (10) à une valeur nominale définie, la passe d'alimentation suivant la passe d'extraction.

5. Procédé selon l'une ou plusieurs des revendications 1 à 3, **caractérisé en ce qu'**en régime permanent, la matière fermentée (5) est extraite de manière strictement continue et la matière organique est introduite de manière strictement continue, chaque fois à un débit qui correspond à un temps de séjour ($\theta$) de la masse en fermentation (4) dans la cuve de fermentation (10), de manière à maintenir constants le poids ou le niveau de la masse en fermentation (4) dans la cuve de fermentation (10).

6. Procédé selon l'une ou plusieurs des revendications 1 à 3, **caractérisé en ce que** la matière organique (1) est traitée par cycles successifs dans chacun desquels la matière organique (1) est introduite en une fois dans la cuve de fermentation (10), y est maintenue en fermentation pendant une durée qui correspond à un temps de séjour ($\theta$) et est extraite en une fois de la cuve de fermentation (10).

7. Installation de fermentation anaérobie de matière organique (1) à haute teneur en solides,
**caractérisée en ce qu'**elle comprend comme composants essentiels :

au moins une cuve de fermentation (10) constituée d'une virole (11) en un matériau étanche aux liquides et aux gaz, apte à résister aux contraintes mécaniques, chimiques et biologiques exercées par le contenu de la cuve de fermentation (10), par exemple en acier, de section transversale circulaire ou polygonale et de préférence circulaire, dont la hauteur (H) représente de préférence de l'ordre de 3 à 6 fois le diamètre (D) du cercle inscrit à la section transversale intérieure, posée sur pieds (14) et fermée dans le bas par un cône (12) et dans le haut par un dôme (13), le cône (12) et le dôme (13) étant réalisés en le même matériau que la virole ou en des matériaux différents,
pour chaque cuve de fermentation (10), un système d'extraction (17) qui extrait la matière fermentée (5) et un système d'alimentation (18) qui introduit dans la cuve de fermentation (10) la matière organique (1) à traiter,
pour chaque cuve de fermentation, un système (19) de brassage par le gaz produit (6) essentiellement constitué de la tubulure (191) de sortie du gaz produit (6), d'une vanne de fermeture/ouverture (192), d'une sonde de pression (193) disposée en amont de la vanne (192) dans la direction de sortie du gaz produit (6), d'une sonde de pression (193) disposée en aval de la vanne (192) dans la direction de sortie du gaz produit (6) et d'un régulateur de pression (194) dans lequel on entre des valeurs ($p_s$, $p_i$) de pression et qui ouvre la vanne (192) normalement fermée lorsque la pression mesurée par la sonde de pression (193) située en amont de la vanne (192) atteint ladite valeur haute ($p_s$) de pression et la referme lorsque cette pression est redescendue à la valeur basse ($p_i$) mesurée par la sonde de pression (193) disposée en aval de la vanne (192).

8. Installation de fermentation anaérobie de matière organique (1) à haute teneur en solides selon la revendication 7, **caractérisée en ce que** le système (19) de brassage par le gaz produit (6) ne présente qu'une seule sonde de pression (193) dont les signaux de pression envoyés au régulateur de pression (194) amènent ce dernier à ouvrir la vanne (192) lorsque la pression dans le digesteur atteint une valeur haute ($p_s$) prédéterminée et réglable et à la fermer lorsque cette pression atteint une valeur basse ($p_i$) prédéterminée et/ou réglable.

9. Installation de fermentation anaérobie de matière organique (1) à haute teneur en solides selon la revendication 7, **caractérisée en ce que** le système (19) de brassage par le gaz produit (6) est essentiellement constitué d'une vanne magnétique (192) qui reste fermée sous l'action d'un aimant (195), qui s'ouvre lorsque la pression dans le digesteur atteint une valeur ($p_s$) prédéterminée et réglable, qui reste ouverte sous l'effet du débit de gaz sortant (6) et qui se referme lorsque ce débit est descendu à une valeur ($p_i$) prédéterminée et réglable.

10. Installation de fermentation anaérobie de matière organique (1) à haute teneur en solides selon l'une quelconque des revendications 7 à 9, **caractérisée en ce que** la première pression prédéterminée ($p_s$) est de préférence comprise entre 200 et 1 000 mbar et **en ce que** la deuxième pression prédéterminée ($p_i$) est de préférence égale à la pression qui règne dans une réserve de gaz, ballon ou gazomètre, située en aval dans la direction d'écoulement du gaz produit (6).

11. Installation de fermentation anaérobie de matière organique (1) à haute teneur en solides selon la revendication 7, **caractérisée en ce que** plusieurs cuves de fermentation (10) sont raccordées en parallèle entre une extraction commune de matière fermentée (5) et une alimentation commune en matière organique (1) et sont toutes raccordées à une réserve commune de gaz produit (6) par leur conduit (191) de sortie de gaz et leur système (19) de brassage par le gaz produit (6).

**Patentansprüche**

1. Verfahren zur anaeroben Gärung von organischem Material mit hohem Feststoffanteil durch anaerobe Gärung des organischen Materials in einem gegenüber der Umgebungsluft dichten Gärungsbehälter (10), bei Schichtung und vertikalem Fortschreiten von oben nach unten in dem Gärungsbehälter (10) unter Einwirkung der Schwerkraft, **dadurch gekennzeichnet, dass**
die gärende Masse (4) abwechselnd durch Aufbau des Druckes des erzeugten Gases (6) und plötzliche Entlastung des Druckes des erzeugten Gases (6) durchgemischt wird und
dieser Wechsel durch getaktete Entnahme des erzeugten Gases (6) erzielt wird.

2. Verfahren nach Anspruch 1, **dadurch gekennzeichnet, dass** der Feststoffanteil des organischen Materials (1) vorzugsweise zwischen 15 Gew.-% und 50 Gew.-% liegt.

3. Verfahren nach Anspruch 1, **dadurch gekennzeichnet, dass** das organische Material (1) bei einer Temperatur vergoren wird, die vorzugsweise in einem Bereich von 35 bis 40 °C oder in einem Bereich von 55 bis 65 °C liegt.

4. Verfahren nach einem oder mehreren der Ansprüche 1 bis 3, **dadurch gekennzeichnet, dass** im Dauerbetrieb eine Menge des vergorenen Materials (5), die der in dem Gärungsbehälter enthaltenen Masse geteilt durch die Verweilzeit ($\theta$) der gärenden Masse (4) in dem Gärungsbehälter (10) entspricht, jeden Tag in einem oder mehreren Entnahmedurchgängen aus dem Gärungsbehälter (10) entnommen wird, wobei das Gewicht der Menge an organischem Material (1), das bei jedem Speisungsvorgang eingebracht wird, derart ist, dass es den Füllstand bzw. das Gewicht der in dem Gärungsbehälter (10) enthaltenen Materialmenge auf einen bestimmten Nennwert zurückführt, wobei der Speisungsvorgang auf den Entnahmevorgang folgt.

5. Verfahren nach einem oder mehreren des Ansprüche 1 bis 3, **dadurch gekennzeichnet, dass** im Dauerbetrieb das vergorene Material (5) streng kontinuierlich entnommen wird und das organische Material streng kontinuierlich eingebracht wird jeweils mit einer Durchflussrate, die einer Verweilzeit ($\theta$) der gärenden Masse (4) in dem Gärungsbehälter (10) entspricht, so dass das Gewicht bzw. der Füllstand der gärenden Masse (4) in dem Gärungsbehälter (10) konstant gehalten werden.

6. Verfahren nach einem oder mehreren des Ansprüche 1 bis 3, **dadurch gekennzeichnet, dass** das organische Material (1) in sukzessiven Zyklen behandelt wird, in denen jeweils das organische Material (1) mit einem Mal in den Gärungsbehälter (10) eingebracht wird, darin für eine einer Verweilzeit ($\theta$) entsprechenden Zeitdauer in Gärung gehalten wird und mit einem Mal aus dem Gärungsbehälter (10) entnommen wird.

**7.** Anlage für die anaerobe Gärung von organischem Material (1) mit hohem Feststoffanteil, **dadurch gekennzeichnet, dass** sie als wesentliche Komponenten enthält:

mindestens einen Gärungsbehälter (10), der aus einem Mantel (11) aus einem flüssigkeits- und gasdichten Material, das geeignet ist, den mechanischen, chemischen und biologischen Beanspruchungen durch den Inhalt des Gärungsbehälters (10) standzuhalten, beispielsweise aus Stahlt besteht, der einen kreisförmigen oder mehreckigen, vorzugsweise kreisförmigen Querschnitt aufweist, dessen Höhe (H) vorzugsweise in der Größenordnung des Drei- bis Sechsfachen des Durchmessers (D) des Kreises liegt, der dem Innenquerschnitt einbeschrieben ist, der auf Füßen (14) steht und unten durch einen Konus (12) und oben durch einen Dom (13) verschlossen ist, wobei der Konus (12) und der Dom (13) aus demselben Material wie der Mantel bestehen oder aus unterschiedlichen Materialien,

für jeden Gärungsbehälter (10) ein Entnahmesystem (17), das das vergorene Material (5) entnimmt, und ein Speisungssystem (18), das das zu behandelnde organische Material (1) in den Gärungsbehälter (10) einbringt, für jeden Gärungsbehälter ein System (19) zum Durchmischen durch das erzeugte Gas (6), welches im Wesentlichen durch den Austrittsstutzen (191) für das erzeugte Gas (6), durch ein Schließ-/Öffnungsventil (192), durch einen vor dem Ventil (192) in Richtung zum Austritt des erzeugten Gases (6) angeordneten Drucksensor (193), durch einen nach dem Ventil (192) in Richtung des Austritts des erzeugten Gases (6) angeordneten Drucksensor (193) und durch einen Druckregulierer (194) gebildet ist, in dem Druckwerte ($p_s$, $p_i$) eingegeben werden und der das normalerweise geschlossene Ventil (192) öffnet, wenn der durch den vor dem Ventil (192) angeordneten Drucksensor (193) gemessene Druck den oberen Druckwert ($p_s$) erreicht und es wieder schließt, wenn dieser Druck auf den unteren Wert ($p_i$) zurückgefallen ist, der durch den nach dem Ventil (192) angeordneten Drucksensor (193) gemessen wird.

**8.** Anlage für die anaerobe Gärung von organischem Material (1) mit hohem Feststoffanteil nach Anspruch 7, **dadurch gekennzeichnet, dass** das System (19) zum Durchmischen durch das erzeugte Gas (6) einen einzigen Drucksensor (193) aufweist, dessen Drucksignale, die an den Druckregulierer (194) gesendet werden, diesen dazu veranlassen, das Ventil (192) zu öffnen, wenn der Druck in dem Faulbehälter einen vorgegebenen und einstellbaren oberen Wert ($p_s$) erreicht und es zu schließen, wenn dieser Druck einen vorgegebenen und/oder einstellbaren unteren Wert ($p_i$) erreicht.

**9.** Anlage für die anaerobe Gärung von organischem Material (1) mit hohem Feststoffanteil nach Anspruch 7, **dadurch gekennzeichnet, dass** das System (19) zum Durchmischen durch das erzeugte Gas (6) im Wesentlichen durch ein Magnetventil (192) gebildet ist, das unter der Einwirkung eines Magneten (195) geschlossen bleibt, das sich öffnet, wenn der Druck in dem Gärungsbehälter einen vorgegebenen und einstellbaren Wert ($p_s$) erreicht, das unter der Einwirkung des austretenden Gasdurchflusses (6) offen bleibt und das sich wieder schließt, wenn dieser Durchfluss auf einen vorgegebenen und einstellbaren Wert ($p_i$) gesunken ist.

**10.** Anlage für die anaerobe Gärung von organischem Material (1) mit hohem Feststoffanteil nach einem beliebigen der Ansprüche 7 bis 9, **dadurch gekennzeichnet, dass** der erste vorgegebene Druck ($p_s$) vorzugsweise zwischen 200 und 1.000 mbar liegt und dass der zweite vorgegebene Druck ($p_i$) vorzugsweise dem Druck entspricht, der in einem in Richtung der Strömung des erzeugten Gases (6) nachgeordneten Gasspeicher, Ballon oder Gasbehälter herrscht.

**11.** Anlage für die anaerobe Gärung von organischem Material (1) mit hohem Feststoffanteil nach Anspruch 7, **dadurch gekennzeichnet, dass** mehrere Gärungsbehälter (10) zwischen einer gemeinsamen Entnahme von vergorenem Material (5) und einer gemeinsamen Speisung mit organischem Material (1) parallel geschaltet sind und alle an einen gemeinsamen Speicher für erzeugtes Gas durch ihre Gasaustrittsleitung (191) und durch ihr System (19) zum Durchmischen durch das erzeugte Gas (6) angeschlossen sind.

**Claims**

**1.** A method for anaerobic fermentation of organic material with a high solids content making use of anaerobic fermentation of the organic material in a fermentation tank (10) sealed from the ambient air, under conditions of stratification and top down vertical feed in the fermentation tank (10) under the action of the gravity,
**characterised in that**
the fermenting mass (4) is agitated by alternately increasing the pressure of the product gas (6) and abruptly relieving the pressure of the product gas (6) and
**in that** said alternation is achieved by cyclically drawing off the product gas (6).

2. A method according to claim 1, **characterised in that** the solids content of the organic material (1) is preferably between 15 and 50% by weight.

3. A method according to claim 1, **characterised in that** the organic material (1) is fermented at a temperature which is preferably in a range from 35 to 40°C or in a range from 55 to 65°C.

4. A method according to one or more of claims 1 to 3, **characterised in that** in steady-state operation a quantity of fermented material (5) equal to the mass present in the fermentation tank divided by the residence time (8) of the fermenting mass (4) in the fermentation tank (10) is removed each day from the fermentation tank (10) in one or more removal operations, the weight of the quantity of organic material (1) during each feed operation being such that it returns the level or weight of the quantity of material present in the fermentation tank (10) to a defined nominal value, the feed operation following the removal operation.

5. A method according to one or more of claims 1 to 3, **characterised in that** in steady-state operation the fermented material (5) is removed in a strictly continuous manner and the organic material is introduced in a strictly continuous manner, in each case at a flow rate which corresponds to a residence time (9) of the fermenting mass (4) in the fermentation tank (10) so as to maintain a constant weight or level of the fermenting mass (4) in the fermentation tank (10).

6. A method according to one or more of claims 1 to 3, **characterised in that** the organic material (1) is processed in successive cycles in each of which the organic material (1) is introduced into the fermentation tank (10) in one go, is kept fermenting there for a period which corresponds to a residence time (9) and is removed from the fermentation tank (10) in one go.

7. An installation for anaerobic fermentation of organic material (1) with a high solids content, **characterised in that** it comprises as essential components:

at least one fermentation tank (10) made up of a shell (11) made from a liquid- and gas-tight material capable of withstanding the mechanical, chemical and biological stresses exerted by the contents of the fermentation tank (10), for example made from steel, of circular or polygonal cross-section and preferably of circular cross-section, the height (H) of which is preferably of the order of 3 to 6 times the diameter (D) of the incircle in the internal cross-section, mounted on feet (14) and closed at the bottom by a cone (12) and at the top by a dome (13), the cone (12) and dome (13) being made from the same material as the shell or from different materials,
for each fermentation tank (10), a removal system (17) which removes the fermented material (5) and a feed system (18) which introduces the organic material (1) to be processed into the fermentation tank (10),
for each fermentation tank, a system (19) for agitation by the product gas (6) essentially made up of the product gas (6) outlet tubing (191), a closing/opening valve (192), a pressure sensor (193) arranged upstream of the valve (192) in the product gas (6) outlet direction, a pressure sensor (193) arranged downstream of the valve (192) in the product gas (6) outlet direction and a pressure controller (194) into which pressure values ($p_s$, $p_i$) are entered and which opens the normally closed valve (192) when the pressure measured by the pressure sensor (193) located upstream of the valve (192) reaches said high pressure value ($p_s$) and closes it again when said pressure has dropped back down to the low value ($p_i$) measured by the pressure sensor (193) arranged downstream of the valve (192).

8. An installation for anaerobic fermentation of organic material (1) with a high solids content according to claim 7, **characterised in that** the system (19) for agitation by the product gas (6) has just one pressure sensor (193), the pressure signals from which forwarded to the pressure controller (194) cause the latter to open the valve (192) when the pressure in the digester reaches a predetermined and controllable high value ($p_s$) and to close it when said pressure reaches a predetermined and/or controllable low value ($p_i$).

9. An installation for anaerobic fermentation of organic material (1) with a high solids content according to claim 7, **characterised in that** the system (19) for agitation by the product gas (6) is essentially made up of a solenoid valve (192) which remains closed under the action of a magnet (195), which opens when the pressure in the digester reaches a predetermined and controllable value ($p_s$), which remains open under the action of the flow rate of the outgoing gas (6) and which closes again when said flow rate has dropped to a pressure and controllable value ($p_i$).

10. An installation for anaerobic fermentation of organic material (1) with a high solids content according to any one of claims 7 to 9, **characterised in that** the first predetermined pressure ($p_s$) is preferably between 200 and 1,000 mbar and **in that** the second predetermined pressure ($p_i$) is preferably equal to the pressure which prevails in a gas store, reservoir or gasometer located downstream in the direction of flow of the product gas (6).

11. An installation for anaerobic fermentation of organic material (1) with a high solids content according to claim 7, **characterised in that** a plurality of fermentation tanks (10) are connected in parallel between a common fermented material (5) removal means and a common organic material (1) feed means and are all connected to a common product gas (6) store via their respective gas outlet ducts (191) and systems (19) for agitation by the product gas (6).

Figure 1

Figure 2

Figure 3

Figure 4

Figure 5

Figure 6

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4735724 A
- EP 1397482 B1
- EP 1930404 A1